# EUROPEAN PATENT SPECIFICATION

(11) **EP 1 943 942 B1**
(45) Date of publication and mention of the grant of the patent: **06.01.2010**
(21) Application number: 07000728.1
(22) Date of filing: 15.01.2007
(51) Int. Cl.: A61B 5/00

(54) **Examining device for examining an aortic valve and a pulmonary valve competency**
Vorrichtung zur Prüfung der Tüchtigkeit einer Aortenklappe und einer Pulmonalklappe
Dispositif servant à examiner une valvule aortique et la compétence d'une valvule pulmonaire

(43) Date of publication of application: 16.07.2008
(73) Proprietor: Chang, Jen-Ping, Niaosong Hsiang Kaohsiung Hsien (TW)
(72) Inventor: Chang, Jen-Ping, Kaohsiung Hsien (TW); Tseng, Chi-Nan, North Dist. Tainan City (TW); Liao, Yuh-Der, Gushan Dist. Kaohsiung (TW)
(74) Representative: LOUIS, PÖHLAU, LOHRENTZ

(56) References cited:
- FR-A- 1 314 158
- FR-A1- 2 776 911
- US-A- 3 054 396
- US-A- 5 935 120

## Description

The present invention relates to an examining device for examining an aortic valve and a pulmonary valve competency during a reparative valve surgery.

A human heart has four chambers and four valves. The chambers include a right atrium, a right ventricle, a left atrium and a left ventricle. The valves are a tricuspid valve, a pulmonary valve, a mitral valve and an aortic valve. The tricuspid valve is located between the right atrium and the right ventricle. The pulmonary valve is located between the right ventricle and the main pulmonary artery. The mitral valve is located between the left atrium and the left ventricle. The aortic valve is located between the left ventricle and the aorta. The great vessel means aorta or main pulmonary artery. As blood leaves each chamber, the blood passes through a valve. If the valve is normal and is competent, the valve will make sure the blood flows only in a forward direction. However, if the valve is abnormal and is incompetent, the blood will flow backwards (regurgitation) and cause heart murmur, angina pectoris, dyspnea, cardiomegaly, heart failure or even death.

When a patient's aortic valve or pulmonary valve is diseased, the aortic valve or the pulmonary valve can be replaced by a prosthetic valve or the patient can take a reparative procedures to repair the valve. However, results of reparative procedures are superior to results of replacements. An open heart operation is performed under extracorporeal circulation and cardioplegic cardiac arrest. In general, a valve competency after a reparative surgery is unable to be confirmed with echocardiography during the cardioplegic cardiac arrest, unless the heart is reperfused and heartbeats are restored. Therefore, if the valvular incompetency is detected after the operation, a second operation under repeated cardioplegic cardiac arrest is mandatory and this carries significant risks.

French patent application FR 2 776 911 discloses an examining device for examining an aortic valve and a pulmonary valve having: a cylinder having a central hole defined axially through the cylinder; a proximal end having a first opening defined through proximal end of the cylinder and communicating with the central hole; and a transparent window mounted on the proximal end and sealing the first opening; a distal end having a second opening defined through the distal end and communicating with the first opening of the proximal end and the central hole; a liquid channel defined through the cylinder and comprising a liquid intake hole formed in the proximal end of the cylinder; and a liquid outtake hole communicating with the liquid intake hole; and an air channel defined through the cylinder and comprising a first air intake hole; and an air outtake hole defined through the proximal end of the cylinder and communicating with the air intake hole; and a liquid infusion catheter assembly connecting with the proximal end of the cylinder.

The primary objective of the present invention is to provide an examining device for examining an aortic valve and a pulmonary valve competency during cardioplegic cardiac arrest for a reparative valvular surgery.

To achieve the objective, the examining device for examining an aortic valve and a pulmonary valve contents a cylindrical structure with 2 build-in channels and a liquid infusion catheter assembly, wherein one of the 2 build-in channels is for liquid infusion and another is for air venting. The cylindrical structure has a proximal end, a distal end, a liquid channel and an air channel. The proximal end has a first opening and a transparent lens. The first opening of the cylindrical structure is defined through the proximal end of the cylindrical structure. The transparent lens is mounted on the proximal end and seals the first opening. A distal end has a second opening. The second opening is defined through the distal end and communicates with the first opening. The liquid channel has a liquid intake hole and a liquid outtake hole. The liquid intake hole is formed in the proximal end. The liquid outtake hole is formed in the distal end and communicates with the liquid intake hole. The air channel has a first air intake hole and an air outtake hole. The first air intake hole is defined through the distal end. The air outtake hole is defined through the proximal end and communicates with the air intake hole. The liquid infusion catheter assembly connects with the proximal end of the cylinder. Moreover, the cylinder further has an outer surface comprising an autoinflatable balloon made of silica gel, mounted on the outer surface and covering the outer surface. The liquid channel further comprises an inside liquid outtake hole formed in the outer surface and communicating with the liquid intake hole and the autoinflatable balloon; and an inside liquid intake hole formed in the outer surface, communicating with the liquid outtake hole and the autoinflatable balloon. After finishing the reparative valvular procedure, surgeons can examine whether the surgery is success or not by using the examining device during the cardiac arrest. If the procedure is failed, surgeon can deal with problems at once, so operative risks can be minimized.

### IN THE DRAWINGS :

Fig. 1 is an operational view in partial section of an examining device for examining an aortic valve and a pulmonary valve in accordance with the present invention mounted in a great vessel.
Fig. 2 is an enlarged cross sectional side view of the examining device for examining the aortic valve and the pulmonary valve in Fig. 1 without a liquid infusion catheter assembly and an air venting catheter assembly;
Fig. 3 is a perspective view of the examining device for examining the aortic valve and the pulmonary valve in Fig. 2 without an autoinflatable balloon;
Fig. 4 is a perspective view of the examining device for examining the aortic valve and the pulmonary valve in Fig. 2; and
Fig. 5 is an operational side view in partial section of the examining device for examining the aortic valve and the pulmonary valve in Fig. 1 after liquid flows into the space through a liquid infusion catheter assembly; and
Fig. 6 is an operational view in partial section of the examining device for examining the aortic valve and the pulmonary valve in Fig. 1 mounted in the great vessel and out of the aortic valve or the pulmonary valve.

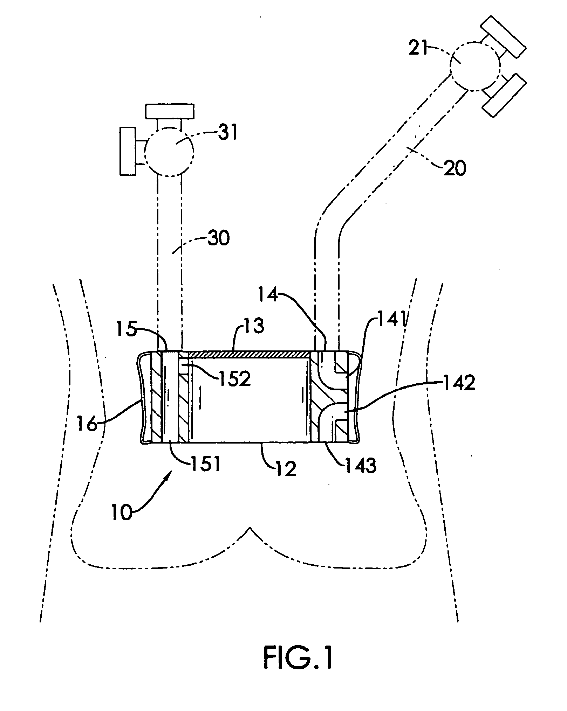

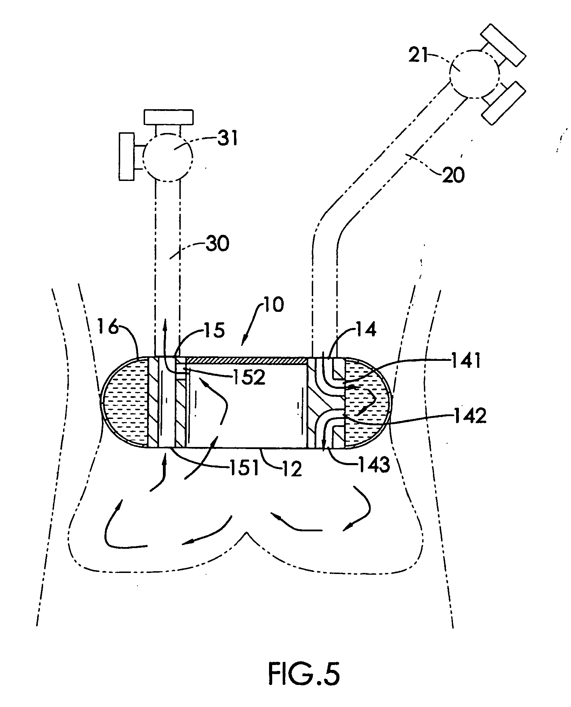

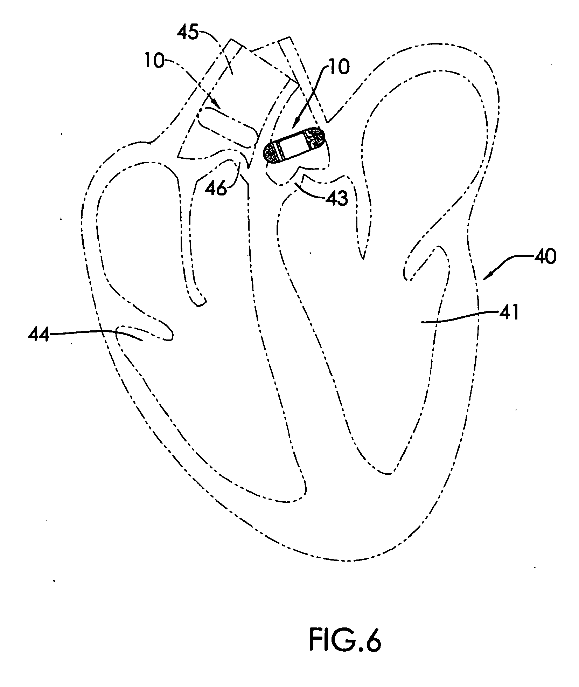

With reference to Figs. 1, 5 and 6, an examining device for examining an aortic valve (43) and a pulmonary valve (46) in accordance with the present invention is mounted in the aortic (42) or the pulmonary root (45) and above the valve (43, 46). A space is formed between the valve (43, 46) and the examining device. The examining device has a hollow cylinder (10), a liquid infusion catheter assembly and an air venting catheter assembly.

The cylinder (10) has a central hole, a proximal end, a distal end, an outer surface, an inner surface, a liquid channel and an air channel.

The central hole is defined axially through the cylinder (10).

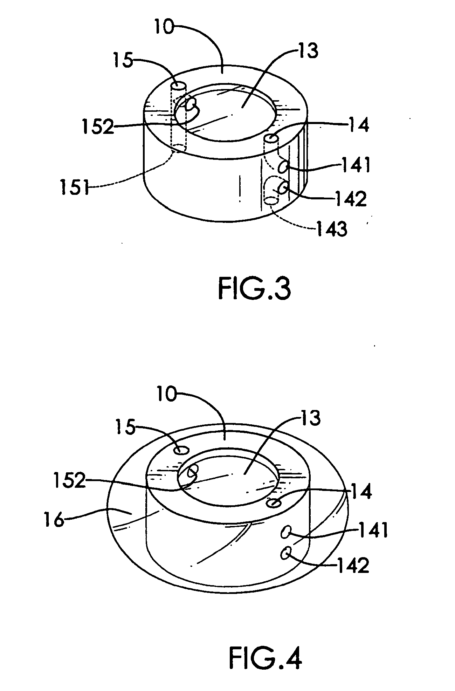

With further reference to Fig. 3, the proximal end has a first opening, a transparent lens (13), a liquid intake hole (14) and an air outtake hole (15). The first opening is defined through proximal end of the cylinder (10) and communicates with the central hole. The transparent lens (13) is mounted on the proximal end and seals the first opening.

The distal end has a second opening (12), a liquid outtake hole (143) and a first air intake hole (151). The second opening (12) is defined through the distal end and communicates with the first opening of the proximal end and the central hole.

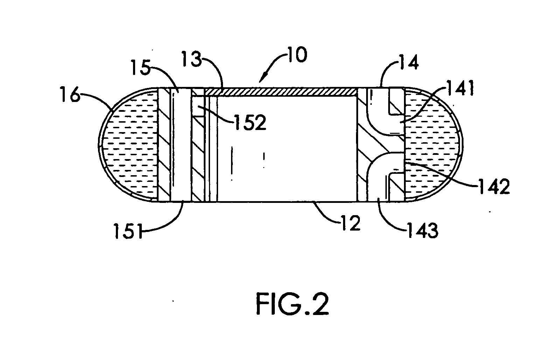

With further reference to Figs. 2 and 4, the outer surface has an autoinflatable balloon (16). The autoinflatable balloon (16) is made of silica gel, is mounted on the outer surface and covers the outer surface.

The liquid channel is defined through the cylinder (10) and comprises a liquid intake hole (14) and a liquid outtake hole (143). The liquid intake hole (14) is formed in the proximal end of the cylinder (10) and allow liquid to flow through the cylinder (10) into the space. The liquid outtake hole (143) is formed in the distal end of the cylinder (10) and communicates with the liquid intake hole (14). The liquid channel may have a first bypass and a second bypass to allow liquid flow into and expand the autoinflatable balloon (16). The first bypass has an inside liquid outtake hole (141). The inside liquid outtake hole (141) is formed in the outer surface, communicates with the liquid intake hole (14) and allows liquid to flow in the autoinflatable balloon (16) from the liquid intake hole (14) and the inside liquid outtake hole (141). Thus, when the autoinflatable balloon (16) filled with liquid, the autoinflatable balloon (16) will expand and abut a great vessels including the aortic (42) and the pulmonary root (45), so the examining device can be mounted in the great vessels (42, 45) securely. The second bypass has an inside liquid intake hole (142). The inside liquid intake hole (142) is formed in the outer surface, communicates with the liquid outtake hole (143) and the autoinflatable balloon (16) and allows liquid in the autoinflatable balloon (16) to flow into the space between the examining device and the valve (43, 46) through the inside liquid intake hole (142) and the liquid outtake hole (143). Thus, when the space is filled with liquid, surgeons can observe a morphology of the valve leaflets from the transparent lens (13) whether the liquid is leaking or not. If the liquid in the space is leaking, the valve (43, 46) is incompetent. If the liquid in the space is not leaking, the valve (43, 46) is competent.

The air channel is defined through the cylinder (10) and has a first air intake hole (151), an air outtake hole (15) and a second air outtake hole (152). The first air intake hole (151) is defined through the distal end. The air outtake hole (15) is defined through the proximal end of the cylinder (10), communicates with the air intake hole (151) and allows air between the valve (43, 46) and the examining device to flow from the first air intake hole (151) to the air outtake hole (15). The inner surface has a second air outtake hole (152). The second air outtake hole (152) is formed in the inner surface of the cylinder (10) adjacent to the proximal end of the cylinder (10) and communicates with the air outtake hole (15). Air in the space can be released completely through the second air outtake hole (152). The liquid infusion catheter assembly connects with the proximal end of the cylinder (10) and has a catheter (20) and a 3-way occluder (21). The catheter (20) connects to and communicates with the liquid intake hole (14) and allows liquid to flow in the cylinder (10) from the catheter (20). The 3-way occluder (21) is mounted on the catheter (20) and controls a flow rate of the liquid in the catheter (20).

The air venting catheter assembly connects with the proximal end of the cylinder (10) and has a rigid catheter (30) and a 3-way occluder (31). The catheter (30) connects to and communicates with the air outtake hole (15), allows air to release out of the cylinder (10) through the catheter (30) and allows a surgeon to hold the examining device. The 3-way occluder (31) is mounted on the catheter (30) and controls a flow rate of the air in the catheter (30).

After completing the procedures for repairing a patient's aortic or pulmonary valve and before reperfusion, surgeons can examine whether the surgery is success or not by using the examining device during the cardioplegic cardiac arrest. If the repair is failed, surgeon can deal with problems at once, so the patient can avoid the risk of the repeated cardioplegic cardiac arrest.

## Claims

1. An examining device for examining an aortic valve (43) and a pulmonary valve (46), and the examining device having
a cylinder (10) having
a central hole defined axially through the cylinder (10);
a proximal end having
a first opening defined through proximal end of the cylinder (10) and communicating with the central hole; and
a transparent lens (13) mounted on the proximal end and sealing the first opening;
a distal end having
a second opening (12) defined through the distal end and communicating with the first opening of the proximal end and the central hole;
a liquid channel defined through the cylinder (10) and comprising
a liquid intake hole (14) formed in the proximal end of the cylinder (10); and
a liquid outtake hole (143) formed in the distal end of the cylinder (10) and communicating with the liquid intake hole (14); and
an air channel defined through the cylinder (10) and comprising
a first air intake hole (151) defined through the distal end; and
an air outtake hole (15) defined through the proximal end of the cylinder (10) and communicating with the air intake hole (151); and
a liquid infusion catheter assembly connecting with the proximal end of the cylinder (10);
wherein
the cylinder (10) further has an outer surface comprising an autoinflatable balloon (16) made of silica gel, mounted on the outer surface and covering the outer surface; and
the liquid channel further comprises
an inside liquid outtake hole (141) formed in the outer surface and communicating with the liquid intake hole (14) and the autoinflatable balloon (16); and
an inside liquid intake hole (142) formed in the outer surface, communicating with the liquid outtake hole (143) and the autoinflatable balloon (16).

2. The examining device for examining an aortic valve (43) and a pulmonary valve (46) as claimed in claim 1, wherein the cylinder further has an inner surface having a second air outtake hole (152) formed in the inner surface of the cylinder (10) adjacent to the proximal end of the cylinder (10) and communicating with the air outtake hole (15).

3. The examining device for examining an aortic valve (43) and a pulmonary valve (46) as claimed in claim 1, wherein the liquid infusion catheter assembly has
a catheter (20) connecting to and communicating with the liquid intake hole (14); and
a 3-way occluder (21) mounted on the catheter (20) and adapted to control a flow rate of liquid in the catheter (20).

4. The examining device for examining an aortic valve (43) and a pulmonary valve (46) as claimed in claim 1 further having an air venting catheter assembly connecting with the proximal end of the cylinder (10) and having
a rigid catheter (30) connecting to and communicating with the air outtake hole (15); and
a 3-way occluder (31) mounted on the rigid catheter (30) and adapted to control a flow rate of air in the catheter (30).

## Patentansprüche

1. Untersuchungsvorrichtung zum Untersuchen einer Aortenklappe (43) und einer Pulmonalklappe (46), wobei die Untersuchungsvorrichtung umfasst:
einen Zylinder (10) mit
einem mittigen Loch, das axial durch den Zylinder (10) hindurch definiert ist;
einem proximalen Ende, das aufweist:
eine erste Öffnung, die durch das proximale Ende des Zylinders (10) hindurch definiert ist und mit dem mittigen Loch in Verbindung steht; und
eine transparente Linse (13), die am proximalen Ende angebracht ist und die erste Öffnung verschließt;
einem distalen Ende, das aufweist:
eine zweite Öffnung (12), die durch das distale Ende hindurch definiert ist und mit der ersten Öffnung des proximalen Endes sowie mit dem mittigen Loch in Verbindung steht;
einem Flüssigkeitskanal, der durch den Zylinder (10) hindurch definiert ist und aufweist:
ein Flüssigkeitseinlassloch (14), das im proximalen Ende des Zylinders (10) ausgebildet ist; und
ein Flüssigkeitsauslassloch (143), das im distalen Ende des Zylinders (10) ausgebildet ist und mit dem Flüssigkeitseinlassloch (14) in Verbindung steht; und
einem Luftkanal, der durch den Zylinder (10) hindurch definiert ist und aufweist:
ein erstes Lufteinlassloch (151), das durch das distale Ende hindurch definiert ist und;
ein Luftauslassloch (15), das durch das proximale Ende des Zylinders (10) hindurch definiert ist und mit dem Lufteinlassloch (151) in Verbindung steht; und
eine Flüssigkeitsinfusions-Katheteranordnung, die mit dem proximalen Ende des Zylinders (10) verbunden ist;
wobei
der Zylinder (10) ferner eine äußere Oberfläche besitzt, die einen aus Silikagel hergestellten selbsttätig aufblasbaren Ballon (16) aufweist, der an der äußeren Oberfläche angebracht ist und die äußere Oberfläche abdeckt; und
der Flüssigkeitskanal ferner umfasst:
ein inneres Flüssigkeitsauslassloch (141), das in der äußeren Oberfläche ausgebildet ist und mit dem Flüssigkeitseinlassloch (14) sowie mit dem selbsttätig aufblasbaren Ballon (16) in Verbindung steht; und
ein inneres Flüssigkeitseinlassloch (142), das in der äußeren Oberfläche ausgebildet ist und mit dem Flüssigkeitsauslassloch (143) sowie mit dem selbsttätig aufblasbaren Ballon (16) in Verbindung steht.

2. Untersuchungsvorrichtung zum Untersuchen einer Aortenklappe (43) und einer Pulmonalklappe (46) nach Anspruch 1, wobei der Zylinder ferner eine innere Oberfläche aufweist, die ein zweites Luftauslassloch (152) besitzt, das in der inneren Oberfläche des Zylinders (10) in der Nähe des proximalen Endes des Zylinders (10) ausgebildet ist und mit dem Luftauslassloch (15) in Verbindung steht.

3. Untersuchungsvorrichtung zum Untersuchen einer Aortenklappe (43) und einer Pulmonalklappe (46) nach Anspruch 1, wobei die Flüssigkeitsinfusions-Katheteranordnung umfasst:
einen Katheter (20), der mit dem Flüssigkeitseinlassloch (14) verbunden ist und damit kommuniziert; und
einen Dreiwegeverschluss (21), der am Katheter (20) angebracht ist und dazu ausgelegt ist, eine Flüssigkeitsdurchflussmenge in den Katheter (20) zu steuern.

4. Untersuchungsvorrichtung zum Untersuchen einer Aortenklappe (43) und einer Pulmonalklappe (46) nach Anspruch 1, die ferner eine Entlüftungs-Katheteranordnung umfasst, die mit dem proximalen Ende des Zylinders (10) verbunden ist und aufweist:
einen starren Katheter (30), der mit dem Luftauslassloch (15) verbunden ist und damit kommuniziert; und
einen Dreiwegeverschluss (31), der am starren Katheter (30) angebracht ist und dazu ausgelegt ist, eine Luftdurchflussmenge in den Katheter (30) zu steuern.

## Revendications

1. Dispositif d'examen destiné à examiner une valve aortique (43) et une valve pulmonaire (46), le dispositif d'examen comprenant:
un cylindre (10) comportant
un orifice central défini axialement à travers le cylindre (10) ;
une extrémité proximale comportant
une première ouverture définie à travers l'extrémité proximale du cylindre (10) et communiquant avec l'orifice central ; et
un lentille transparente (13) montée sur l'extrémité proximale et assurant l'étanchéité de la première ouverture ;
une extrémité distale comportant
une seconde ouverture (12) définie à travers l'extrémité distale et communiquant avec la première ouverture de l'extrémité proximale et l'orifice central ;
un canal de liquide défini à travers un cylindre (10) et comprenant
un orifice d'entrée de liquide (14) formé sur l'extrémité proximale du cylindre (10) ; et
un orifice de sortie de liquide (143) formé sur l'extrémité distale du cylindre (10) et communiquant avec l'orifice d'entrée de liquide (14) ; et
un canal d'air défini à travers le cylindre (10) et comprenant
un premier orifice d'entrée d'air (151) défini à travers l'extrémité distale ; et
un orifice de sortie d'air (15) défini à travers l'extrémité proximale du cylindre (10) et communiquant avec l'orifice d'entrée d'air (151) ; et
un ensemble cathéter d'administration de liquide relié à l'extrémité proximale du cylindre (10) ;
dans lequel
le cylindre (10) comporte, en outre, une surface externe comprenant un ballonnet auto-gonflable (16) réalisé en un gel de silice, monté sur la surface externe et recouvrant la surface externe ; et
le canal de liquide comprend, en outre :
un orifice de sortie de liquide interne (141) formé sur la surface externe et communiquant avec l'orifice d'entrée de liquide (14) et le ballonnet auto-gonflable (16), et
un orifice d'entrée de liquide interne (142) formé sur la surface externe, communiquant avec l'orifice de sortie de liquide (143) et le ballonnet auto-gonflable (16).

2. Dispositif d'examen destiné à examiner une valve aortique (43) et une valve pulmonaire (46) selon la revendication 1, dans lequel le cylindre comprend, en outre, une surface interne comportant un second orifice de sortie d'air (152) formé sur la surface interne du cylindre (10) de manière adjacente à l'extrémité proximale du cylindre (10) et communiquant avec l'orifice de sortie d'air (15).

3. Dispositif d'examen destiné à examiner une valve aortique (43) et une valve pulmonaire (46) selon la revendication 1, dans lequel l'ensemble de cathéter d'administration de liquide comprend
un cathéter (20) raccordé sur l'orifice d'entrée de liquide (14) et communiquant avec ce dernier ; et
un dispositif d'obturation à 3 voies (21) monté sur le cathéter (20) et adapté afin de commander un débit de liquide dans le cathéter (20).

4. Dispositif d'examen destiné à examiner une valve aortique (43) et une valve pulmonaire (46) selon la revendication 1, comprenant, en outre, un ensemble de cathéter de mise à l'air relié à l'extrémité proximale du cylindre (10) et comportant
un cathéter rigide (30) relié à l'orifice de sortie d'air (15) et communiquant avec ce dernier ; et
un dispositif d'obturation à 3 voies (31) monté sur le cathéter rigide (30) et adapté de manière à commander un débit d'air dans le cathéter (30).
